# EUROPEAN PATENT APPLICATION

(11) **EP 4 483 794 A1**
(43) Date of publication of application: **01.01.2025**
(21) Application number: 23759826.3
(22) Date of filing: 15.02.2023
(51) Int. Cl.: A61B 5/022, A61B 8/04

(54) **ARTERIOVENOUS PRESSURE MEASUREMENT DEVICE AND ARTERIOVENOUS PRESSURE MEASUREMENT METHOD**

(30) Priority: 22.02.2022 JP 2022026161
(71) Applicant: KURUME UNIVERSITY, Kurume-shi Fukuoka 830-0011 (JP)
(72) Inventor: TOMOEDA, Hiroshi, Kurume-shi, Fukuoka 830-0011 (JP)
(74) Representative: v. Bezold & Partner Patentanwälte - PartG mbB
(86) International application number: PCT/JP2023/005290
(87) International publication number: WO 2023/162825

(57) **Abstract**

The arteriovenous pressure measurement device (1) has a bag (40) that transmits ultrasonic waves (U) that have been transmitted from a probe (2) scanning around one direction and that have been reflected and received by the probe (2). The presser (10) is pressed, in a direction where the ultrasonic waves (U) are transmitted, at a contact surface (20a) created on an outer surface of the bag (40) and intersected by a scanning plane (TF) made by scanning of the ultrasonic waves (U) by the probe (2), wherein the pressing force of the presser is adjustable manually or by an adjustment mechanism. The pressure detector (11) is set in a narrower area than the contact surface (20a) on or directly below the contact surface (20a) and detects pressure applied to the area as arteriovenous pressure under the skin that the area contacts.

## Description

### Technical Field

The present disclosure relates to an arteriovenous pressure measurement device and a method for measuring arteriovenous pressure.

### Background Art

Vascular pressure (arterial and venous pressure) is, for example, an indicator for determining excess or deficiency of a circulating blood volume and for determining the course of treatment by determining right heart failure in the emergency and intensive care fields. Vascular pressure measurement is performed in various medical fields, including the vascular surgery field and the dermatology field. Measurement of venous pressure in the lower extremities in a patient with varicose veins is one example.

Accurate measurement of vascular pressure in the medical field is generally performed using an invasive blood pressure (IBP) monitoring method (refer to Patent Literature 1). In the IBP method, after puncturing a blood vessel at a measurement site and introducing and guiding a catheter, the drip route to the introduced needle or catheter is required to be filled with a liquid and attached to a pressure sensor. Since these operations are time-consuming, using this method in the emergency medical treatment field that requires urgent care may cause a delay in treatment.

In addition, this method is highly invasive to the patient, as it involves vascular puncture and catheter introduction. As such, this method may cause complications such as arteriovenous and cardiac damage, bleeding, bloodstream infection, pneumothorax, and hemothorax. In addition, the patient to be measured is basically tethered to the drip route, making it difficult to move the patient. As a means to overcome these drawbacks, a venous pressure measurement device that uses an ultrasonic measurement probe to measure venous pulse pressure utilizing venous collapse caused by pressing on the skin, without puncturing the blood vessel, has been disclosed (refer to Non Patent Literature 1).

In this venous pressure measurement device, a disk-shaped silicone body is attached to the surface of the probe of the ultrasonic probe. The silicone body is placed against the skin outside a vein to be measured so that the ultrasonic probe is pressed against the skin to collapse the vein. Since the silicone body is designed to transmit ultrasonic waves of the ultrasonic probe, the silicone body does not act as a barrier to the ultrasonic waves, and the ultrasonic probe can acquire an image of the vein in a collapsed state.

A pressure sensor is attached between the ultrasonic probe and the silicone body to measure the pressing force from the ultrasonic probe to the skin. This pressure sensor detects the pressing force of the silicone body when the vein is in a collapsed state as venous pressure.

However, in the venous pressure measurement device disclosed in Non Patent Literature 1, if the vein in the area where venous pressure is to be measured is located in an area with large curvature, such as the front of the lower leg or the forearm, the center of the silicone body is compressed and crushed by the large curvature when the disk-shaped silicone body is applied to the area with large curvature. On the other hand, the portion of the silicone body other than the center of the silicone body, which is not in contact with the body surface, is not in contact with the body and floats in the air, making it impossible to accurately measure venous pressure based on the pressing force of the silicone body detected by the pressure sensor.

Since the disc-shaped silicone body in this venous pressure measurement device has a thin wall thickness, the silicone body on the surface of the measurement device may come into direct contact with the ultrasonic probe or the pressure sensor when the measurement device is pressed against the measurement target, which may cause variations in the measurement values. This affects significantly especially when the curvature of the measurement target is large, likely causing inaccurate measurements.

Therefore, a non-intrusive and accurate, non-invasive arteriovenous pressure measurement device has been disclosed (refer to Patent Literature 2). This non-invasive arteriovenous pressure measurement device can measure not only venous pressure but also arterial pressure, which is basically difficult to measure with the venous pressure measurement device of Non Patent Literature 1.

In addition, non-invasive automatic sphygmomanometers are used at home. Such automatic sphygmomanometers measure blood pressure by pressurizing a cuff wrapped around the upper arm of the subject with air and detecting pressure oscillations that occur when blood passes through areas where there is a difference in caliber between the crushed and uncrushed portions of the artery when the artery is crushed.

In other words, such automatic sphygmomanometers use an oscillometric method to estimate systolic and diastolic blood pressures by auscultation. It has been pointed out that the oscillometric method has a large error in blood pressure measurement. This is because the pulse pressure characteristic ratios that are the basis of the estimation do not correspond exactly to the systolic and diastolic blood pressures by auscultation (refer to Non Patent Literature 2).

### Citation List

### Patent Literature

Patent Literature 1: Unexamined Japanese Patent Application Publication No. 2013-188382
Patent Literature 2: Japanese Patent No. 6718177

### Non Patent Literature

Non Patent Literature 1: Christoph Thalhammer, and 7 others, "Noninvasive Central Venous Pressure Measurement by Controlled Compression Sonography at the Forearm," Journal of the American College of Cardiology, Elsevier Inc.
Non Patent Literature 2: Masanobu Takada, Health Evaluation and Promotion, 2015, Vol. 42, No. 6, p. 39

### Summary of Invention

### Technical Problem

In a general automatic sphygmomanometer, air is filled into a cuff that is wrapped around an upper arm, and this air pressure is increased to measure the internal pressure of an artery based on the air pressure required to crush the artery to be measured. If the pressure exerted on the artery in this process is always equal to the air pressure in the cuff, a measurement error is considered to be small. However, because the cuff is large and the area compressed by the cuff is wide, it may not always be possible to say that the same pressure as the air pressure in the cuff is applied to the artery to be measured for the following reasons.

First, in the case of a general automatic sphygmomanometer, the position of an artery passing through the interior of the upper arm in the range compressed by the cuff includes both shallow and deep areas from the skin surface. When pressure is applied to crush an artery with the cuff from the skin surface to measure the pressure (blood pressure) of the artery passing through a deep area, the air pressure applied from the skin surface is not easily transmitted to the deep area of the arm, and therefore, even if the internal pressure of the artery is the same, the artery cannot be crushed unless it is compressed with stronger pressure compared to a case of compressing an artery in a shallow area. As a result, the measurement of arterial pressure in a deep area based on the air pressure inside the cuff results in an excessive measurement value with a large measurement error.

On the other hand, there are hard tissues inside an upper arm, such as bones, bony prominences, and tendons attached to the bones, especially near an elbow joint where the bones are not cylindrical but flat and have protrusions and prominences on their surfaces. When the upper arm is compressed with the cuff, the pressure is locally stronger around the site where such hard tissues are prominent than the pressure exerted by the cuff. In addition, it is generally considered that the site where a strong pressure is exerted receives the pressure, resulting in weaker-than-average pressure on the tissues surrounding the site. Thus, a pressure imbalance is made inside the living tissues that are compressed by a large cuff.

When such an internal pressure imbalance is made, the arteries and veins running inside the tissues are not compressed with uniform pressure even when a current large cuff is used and an increase in the air pressure inside the cuff is intended to provide uniform pressure with the air pressure. The arteries and veins to be measured may or may not run over bony prominences or projections depending on the person since the original anatomical travel of these arteries and veins varies from person to person. The size and shape of the bony prominences and projections also vary from person to person. Furthermore, the positional relationship of the arteries and veins to the bony prominence changes with a change in the twisting angle of the forearm in relation to the upper arm. Therefore, when an artery or vein is trapped between a bony prominence or other area and the cuff, it is compressed with locally concentrated pressure, causing the artery or vein to be crushed by the air pressure inside the cuff that is smaller than the real internal pressure of the artery or vein. This leads to underestimate the internal pressure, resulting in a large measurement error.

General automatic sphygmomanometers do not take into account any errors due to such vascular depth or pressure imbalance. It is estimated that 60% of arterial pressures measured by automatic sphygmomanometers or mercury column sphygmomanometers fall within ±10 mmHg of the catheter-measured intra-aortic pressure, and 78% fall within ±15 mmHg.

An objective of the present disclosure is to provide an arteriovenous pressure measurement device and a method for measuring arteriovenous pressure that can measure arteriovenous pressure non-invasively and more accurately.

### Solution to Problem

In order to achieve the above objective, an arteriovenous pressure measurement device according to a first aspect of the disclosure, is an arteriovenous pressure measurement device including:
a presser that includes a member that transmits ultrasonic waves that have been transmitted from a probe scanning around one direction and that have been reflected and received by the probe, wherein the presser is pressed, in a direction where the ultrasonic waves are transmitted, at a contact surface created on an outer surface of the member and intersected by a scanning plane made by scanning of the ultrasonic waves by the probe, wherein the pressing force of the presser is adjustable manually or by an adjustment mechanism; and
a pressure detector that is set in a narrower area than the contact surface on or directly below the contact surface and detects pressure applied to the area by pressing on a skin as arteriovenous pressure under the skin that the area contacts.

The size of the area may be equivalent to or less than the diameter of a blood vessel under the skin that the area contacts.

The pressure detector may include:
a flexible bag that is set in the area, made of a material transmitting the ultrasonic waves, and filled with a fluid transmitting the ultrasonic waves; and
a sensor that detects internal pressure of the bag as pressure exerted on a blood vessel.

The pressure detector may include a pressure sensor that is embedded in the area of the member directly below the contact surface and detects pressure applied to the area from an opposite direction of a pressing direction of the presser as pressure exerted on a blood vessel.

The pressure detector may be displaced from the scanning plane to one or both sides of a normal direction of the scanning plane.

There may be a plurality of pressure detectors.

The pressure detectors may be arranged in one or more rows.

The presser may include:
a flexible container that is made of a flexible material that transmits the ultrasonic waves and contains a fluid that transmits the ultrasonic waves; and
a housing that includes:
   a first opening that houses the flexible container, wherein the flexible container bulges in a direction of pressing on the skin to create the contact surface; and
   a second opening that opposes the first opening and allows ultrasonic waves transmitted from the probe to enter through the second opening.

The housing may include:
a third opening; and
the presser may include an external manipulator that causes the contact surface of the flexible container to bulge through the first opening when an operator manually presses on the flexible container through the third opening.

The presser may include a pressing mechanism that presses the probe against the flexible container through the second opening to cause the contact surface of the flexible container to bulge through the first opening.

The presser may include:
a feeder capable of supplying the fluid to the flexible container; and
an actuator that causes, through an input operation by an operator, the feeder to supply the fluid to the flexible container to cause the contact surface of the flexible container to bulge.

The flexible container that bulges through the first opening may have a wall thickness that is thinnest at a portion where the pressure detector is provided and gradually thickens toward an edge of the first opening.

The housing may include: an attachment that mounts the probe so that a pressing direction of the presser is aligned with the scanning plane.

The housing may include a gripper to be held by an operator.

The arteriovenous pressure measurement device may include the probe, wherein
the presser includes an elastic member provided at an outlet of the ultrasonic waves of the probe and transmits the ultrasonic waves, and
the pressure detector is embedded in the elastic member.

The arteriovenous pressure measurement device may include:
a wireless communicator that transmits information indicating pressure detected by the pressure detector via a wireless communication; and
a display that receives information transmitted by the wireless communicator and displays the received information.

A method for measuring arteriovenous pressure according to a second aspect of the present disclosure is an arteriovenous pressure measurement method using an arteriovenous pressure measurement device that includes a presser that includes a member that transmits ultrasonic waves that have been transmitted from a probe scanning around one direction and have been reflected and received by the probe, wherein the presser is pressed, in a direction where the ultrasonic waves are transmitted, at a contact surface created on an outer surface of the member and intersected by a scanning plane made by scanning of the ultrasonic waves by the probe, wherein the pressing force of the presser is adjustable manually or by an adjustment mechanism; and a pressure detector that is set in a narrower area than the contact surface on or directly below the contact surface and detects pressure applied to the area by pressing on a skin as arteriovenous pressure under the skin that the area contacts,
the method including:
disposing the arteriovenous pressure measurement device so that the area is in contact with the skin;
initializing pressure to be detected by the pressure detector in a state where the presser is not pressing on the skin;
starting to transmit and receive the ultrasonic waves by the probe;
pressing on the skin by the presser;
displaying a tomographic image of a blood vessel based on the ultrasonic waves received by the probe; and
detecting an arteriovenous pressure exerted on the blood vessel under the skin in a state where the presser is pressing on the skin by the pressure detector.

### Advantageous Effects of Invention

According to the present disclosure, while pressing on the skin with a presser to crush a blood vessel under the skin, the pressure detector can detect not the average pressure applied to the blood vessel and surrounding tissues as a whole but the pressure applied to a narrower area where the skin and the presser are in contact, that is, a pressure value closer to the actual pressure exerted on the blood vessel. This allows non-invasive and more accurate measurement of arteriovenous pressure.

### Brief Description of Drawings

FIG. 1A is a schematic diagram illustrating the appearance of an arteriovenous pressure measurement device according to Embodiment 1 of the present disclosure;
FIG. 1B is a diagram illustrating how a probe is attached to the arteriovenous pressure measurement device of FIG. 1A;
FIG. 1C is a perspective diagram of the arteriovenous pressure measurement device of FIG. 1A with the probe attached;
FIG. 2A is a cross-sectional diagram of the arteriovenous pressure measurement device illustrated in FIG. 1B taken along the line II-II;
FIG. 2B is a schematic diagram illustrating the relationship between the arteriovenous pressure measurement device and the skin, viewed from a different direction from FIG. 2A;
FIG. 3 is a flowchart of the arteriovenous pressure measurement processing;
FIG. 4A is a schematic diagram illustrating the proximity to the skin;
FIG. 4B is a schematic diagram illustrating how the skin is pressed on;
FIG. 4C is a schematic diagram illustrating the relationship between the pressure generated in the presser and the pressure detected by the pressure detector in the case illustrated in FIG. 4B;
FIG. 5 is a schematic cross-sectional diagram of an arteriovenous pressure measurement device according to Embodiment 2 of the present disclosure;
FIG. 6 is a schematic cross-sectional diagram of an arteriovenous pressure measurement device according to Embodiment 3 of the present disclosure;
FIG. 7 is a schematic diagram illustrating an enlarged view of the presser and the pressure detector;
FIG. 8 is a perspective diagram of an arteriovenous pressure measurement device according to Embodiment 4 of the present disclosure;
FIG. 9 is a schematic diagram of the internal structure of the arteriovenous pressure measurement device of FIG. 8;
FIG. 10A is a perspective diagram of an arteriovenous pressure measurement device according to Embodiment 5 of the present disclosure;
FIG. 10B is an expanded diagram of the pressure detector of FIG. 10A;
FIG. 11A is a first schematic diagram illustrating the internal structure of the arteriovenous pressure measurement device of FIG. 10A;
FIG. 11B is a second schematic diagram illustrating the internal structure of the arteriovenous pressure measurement device of FIG. 10A;
FIG. 12A is a perspective diagram of the arteriovenous pressure measurement device according to Embodiment 5 of the present disclosure;
FIG. 12B is a perspective diagram illustrating a first variant of the arteriovenous pressure measurement device of FIG. 12A;
FIG. 12C is a perspective diagram illustrating a second variant of the arteriovenous pressure measurement device of FIG. 12A;
FIG. 12D is a perspective diagram illustrating a third variant of the arteriovenous pressure measurement device of FIG. 12A; and
FIG. 13 is a schematic diagram illustrating an area around the pressure detector of the arteriovenous pressure measurement device of FIG. 12A.

### Description of Embodiments

The following describes embodiments of the present disclosure in detail with reference to the drawings. In each drawing, the same or equivalent components are denoted with the same signs. Using the arteriovenous pressure measurement device of the present embodiments enables the measurement of arterial or venous pressure without puncturing an artery or vein, that is, non-invasively. The arteriovenous pressure measurement device is used with a probe that transmits ultrasonic waves while scanning around one direction and receives the reflected ultrasonic waves.

### Embodiment 1

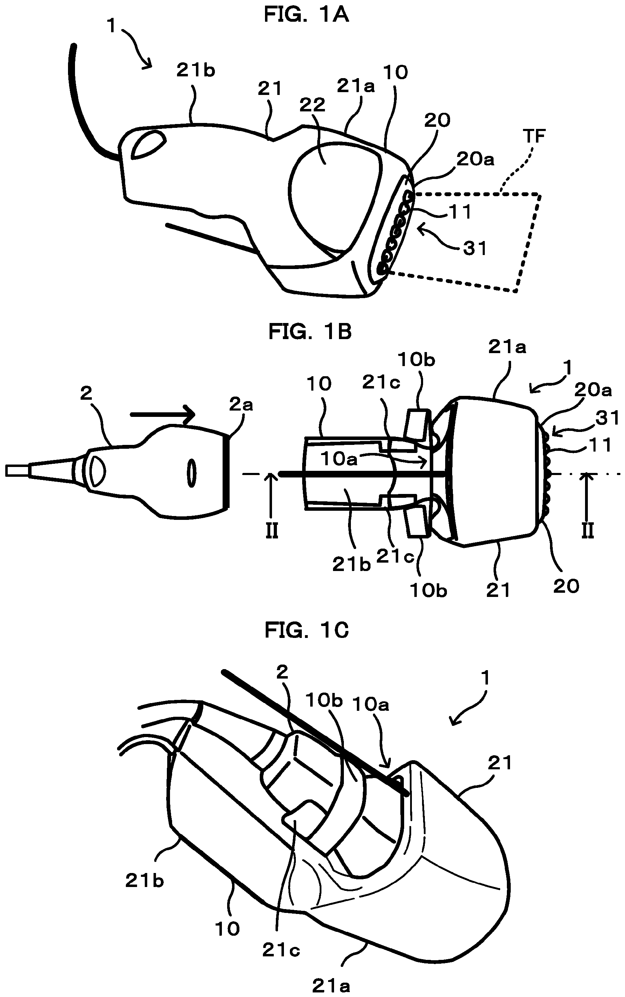

First, Embodiment 1 of the present disclosure is described with reference to FIGS. 1A to 4C. FIG. 1A illustrates the appearance of the arteriovenous pressure measurement device 1 according to the present embodiment. As illustrated in FIG. 1A, the arteriovenous pressure measurement device 1 has a housing 21 as an enclosure.

The housing 21 includes a cover 21a and an extension 21b. The cover 21a houses the main part of the arteriovenous pressure measurement device 1. The extension 21b functions as a gripper to be held by an operator when the arteriovenous pressure measurement device 1 is used.

As illustrated in FIGS. 1B and 1C, the arteriovenous pressure measurement device 1 is used with a probe 2, through which an ultrasonic echo device transmits and receives ultrasonic waves. The cover 21a is provided with a recess 10a, and the extension 21b is provided with a belt 10b in the vicinity of the recess 10a. As illustrated in FIG. 1B, the probe 2 is introduced into the recess 10a with a head 2a that is an ultrasonic transmitting/receiving port facing the recess 10a of the arteriovenous pressure measurement device 1. The housing 21 further includes a pincer piece 21c. The probe 2 introduced into the recess 10a is held by the pincer piece 21c and secured by the belt 10b. The arteriovenous pressure measurement device 1 is used in a state illustrated in FIG. 1C, that is, with the probe 2 attached.

### [Overall structure]

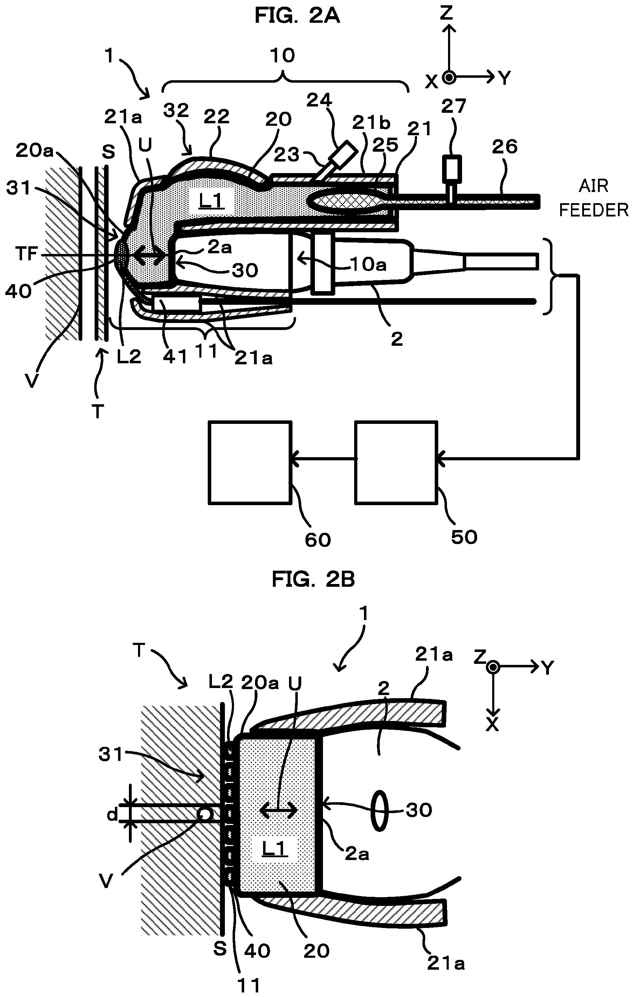

As illustrated in FIG. 2A, the arteriovenous pressure measurement device 1 includes a presser 10 and a pressure detector 11. In FIG. 2A, the running direction of the blood vessel V of the measurement target T is illustrated as a Z-axis direction, the plane along the skin S directly above the blood vessel V is illustrated as an XZ plane, and the direction toward the arteriovenous pressure measurement device 1 viewed from the measurement target T is illustrated as the +Y direction.

### [Presser]

The presser 10 presses on the skin S with a portion that transmits ultrasonic waves U. The presser 10 includes a balloon 20 as a flexible container, a housing 21 as described above as an enclosure, and a push button 22 as an external manipulator. The balloon 20 contains a liquid L1 as a fluid, and the housing 21 houses the balloon 20.

### [Balloon]

The balloon 20 is made of a flexible material that transmits ultrasonic waves U. In other words, balloon 20 is a member that transmits ultrasonic waves U. Such a material includes, for example, silicone resin. However, this material is not limited to silicone resin. Any material that transmits ultrasonic waves U is acceptable. For example, the material can be rubber.

### [Cover]

The housing 21 houses the balloon 20. The cover 21a constituting the housing 21 serves as an attachment where the probe 2 is introduced and mounted in the recess 10a as described above. The cover 21a includes a head opening 30 as a second opening, an echo window 31 as a first opening, and a button attachment opening 32 as a third opening.

Positioned at the head opening 30 is the head 2a of the probe 2 that is introduced into the recess 10a. The head opening 30 allows entering of ultrasonic waves U that are transmitted from the head 2a while scanning is performed in the XY plane around one direction, the -Y direction. The incident ultrasonic waves U travel toward the echo window 31. The head opening 30, in turn, allows entering the reflected ultrasonic waves U that have been reflected at the transmission destination and entered the echo window 31 to the head 2a.

The echo window 31 is positioned opposite the head opening 30 and in contact with the skin S. The balloon 20 bulges through the echo window 31 in the direction of pressing on the skin S, creating a contact surface 20a on the outer surface of the balloon 20 that is in contact with the skin S. The ultrasonic waves U transmitted from the head 2a of the probe 2 pass through the balloon 20 and the liquid L1, and then through the echo window 31 to reach the skin S. In other words, in the echo window 31, the ultrasonic waves U are transmitted in the direction of pressing on the skin S.

The oscillation surface of ultrasonic wave U at the head 2a of the probe 2 is shaped in a circle or ellipse of a size that no part of the plane does not come into contact with the skin, having a periphery of, for example, an ellipse, a rectangle, or a rectangle with rounded four corners. Correspondingly, the head opening 30 and echo window 31 are also shaped to match the shape of the oscillation surface of ultrasonic wave U of this head 2a. The echo window 31 is shaped and sized to allow the entire contact surface 20a of the balloon 20 to contact the skin S. The presser 10 is set on the outer surface of the balloon 20 and is pressed in a direction where ultrasonic waves U are transmitted in the contact surface 20a that bulges through the echo window 31. Even when not being pressed, the contact surface 20a still protrudes from the echo window 31. The button attachment opening 32 is located on the +Z side surface of the cover 21a of the housing 21.

The transmission direction of ultrasonic waves U transmitted from the probe 2 scans in one direction, that is, in the X-axis direction, within a predetermined range. The cover 21a is attached to the probe 2 so that the pressing direction of the presser 10 aligns with the ultrasonic waves U scanning plane (transmission plane) TF (refer to FIG. 1A) created by the scanning by the ultrasonic waves U transmitted from the mounted probe 2 in one direction. As a result, the scanning plane TF created by the ultrasonic waves U scanning by the probe 2 passes through (intersects) the contact surface 20a.

### [Push button]

The push button 22 as an external manipulator is fitted into the button attachment opening 32 as the third opening. The bottom surface of the push button 22 is in contact with the +Z side surface of the balloon 20. The push button 22 press-operates the outer surface (the outer surface corresponding to the button attachment opening 32) of the balloon 20 other than the outer surface of the balloon 20 that bulges through the echo window 31, that is, the portion of the balloon 20 that contacts the skin S (the contact surface 20a). When the push button 22 is manually pressed by the operator from the outside of the housing 21 toward the inside, a portion of the balloon 20 that contacts the push button 22 is concaved, and at the same time, a portion of the outer surface of the balloon 20 other than the concaved portion, that is, the contact surface 20a that bulges through the echo window 31 further bulges outward through the echo window 31 and presses on the skin S with the bulging outer surface (the contact surface 20a). In other words, the presser 10 is pressed in a direction where ultrasonic waves U are transmitted in the contact surface 20a of the outer surface of the balloon 20 that bulges out through the echo window 31. The pressing force can be adjusted by the degree of pressing the push button 22, that is, manually.

### [Adjustment of liquid volume]

The balloon 20 is in communication with the outside through a tube 23. Through the tube 23, the liquid L1 is supplied to and discharged from the balloon 20. A liquid volume adjustment valve 24 is attached to the tube 23. The liquid volume adjustment valve 24 adjusts the supply and discharge volume of the liquid L1 to and from the balloon 20.

The balloon 20 may be provided with an air vent valve (not illustrated). If the air vent valve is opened when injecting the liquid into the balloon 20, the liquid L1 can be injected into the balloon 20 while air in the balloon 20 is driven outward from the air vent valve. This allows the interior of the balloon 20 to be filled only with the liquid L1.

### [Gas bag]

A gas bag 25 as an expandable and contractible bag-like body is disposed inside the balloon 20. By expanding and contracting this gas bag 25, the pressure of the liquid L1 inside the balloon 20 is adjusted. Pressurization of the liquid L1 by the gas bag 25 is effective when the liquid L1 cannot be sufficiently pressurized by the push button 22 alone. In this way, the arteriovenous pressure measurement device 1 of the present embodiment has both the push button 22 and the gas bag 25, but the gas bag 25 may be provided without the push button 22. Alternatively, the push button 22 may be provided without the gas bag 25.

The tube 26 allows air to flow into and out of the gas bag 25; the air expands and contracts the gas bag 25. In the arteriovenous pressure measurement device 1, only the liquid L1 is sealed inside the balloon 20, and there is no air inside the balloon 20. Therefore, the liquid pressure inside the balloon 20 can be increased or decreased by the amount that the gas bag 25 expands or contracts.

In the arteriovenous pressure measurement device 1, air is used as the flow into and out of the gas bag 25, but without limitation, for example, a liquid may also be used. In the case of using a liquid, the liquid volume inside the balloon 20 may be increased or decreased directly without using the gas bag 25.

The tube 26 is also equipped with a pressure adjustment valve 27. The pressure adjustment valve 27 is disposed so that the gas bag 25 is positioned on the back pressure side (+Y side in FIG. 2A).

When the valve of the pressure adjustment valve 27 is opened, high-pressure air from an air supply flows into the gas bag 25, causing the gas bag 25 to inflate. When the valve of the pressure adjustment valve 27 is closed, the gas bag 25 is held in an expanded state. To increase the pressing force on the skin S, the pressure adjustment valve 27 is opened to expand the gas bag 25, thereby expanding the balloon 20. This expansion of the balloon 20 can increase the pressing force on the skin S.

The tube 26 has an outlet, not illustrated, for air to flow out of the gas bag 25. This outlet is located between the gas bag 25 and the pressure adjustment valve 27. An open/close valve is attached to the outlet through a tubing. The open/close valve has one end communicating with the outlet and the other end open. When the open/close valve is opened, air flows out of the gas bag 25, causing the gas bag 25 to contract. When the open/close valve is closed, no air flows out of the gas bag 25, and the pressure at the time of closing the open/close valve is maintained in the gas bag 25. When the pressing force on the skin S is desired to be reduced, the open/close valve is opened and the gas bag 25 is contracted to contract the balloon 20. This contraction of the balloon 20 can reduce the pressing force on the skin S. In any case, the arteriovenous pressure measurement device 1 is equipped with an adjustment mechanism that supplies gas to the gas bag 25 and the liquid L1 into the balloon 20, thus, the pressing force of the balloon 20 can be adjusted by the adjustment mechanism.

### [Pressure detector]

As illustrated in FIG. 2A, at least a portion of the pressure detector 11 is positioned between the presser 10 and the skin S. The pressure detector 11 includes a bag 40 and a sensor 41.

### [Bag]

As illustrated in FIGS. 2A and 2B, the bag 40 is disposed on an area where the skin S above the blood vessel V and the presser 10 are in contact, that is, the contact surface 20a. The bag 40 is set in a narrower area than the contact surface 20a on the contact surface 20a. The bag 40 is made of a flexible material that transmits ultrasonic waves U, like the balloon 20.

The bag 40 has a size equivalent to or less than the diameter of the blood vessel V under the skin S that is pressed by the presser 10. In other words, the size of the area in which the bag 40 is set is equivalent to or less than the diameter of the blood vessel under the skin to be in contact with the area. More specifically, as illustrated in FIG. 2B, in the present embodiment, the width of the bag 40 in the X-axis direction is equivalent to or less than the diameter d of the blood vessel V. The diameter d of the blood vessel V varies among individuals, and even within the same individual, the diameter d varies among measurement sites. The artery and vein to be measured may be any artery or vein throughout the body that can be compressed through the skin surface. Basically, the artery can be the brachial artery, radial artery, dorsal pedal artery, carotid artery, or femoral artery, while the vein can be assumed as the external jugular vein, internal jugular vein, shallow femoral vein, or great saphenous vein. However, the measurement site is not necessarily limited to these arteries and veins. The width in the X-axis direction of the bag 40, which is assumed based on the blood vessel V to be the measurement site, can be 1 mm or less at minimum and several centimeters (3 to 4 cm) at maximum.

The interior of the bag 40 is filled with a liquid L2 as a fluid. The liquid L2 also transmits ultrasonic waves U. The liquid L2 is, for example, water. However, the liquid L2 is not limited to water. The amount of the liquid L2 in the bag 40 may also be adjustable in a similar configuration to that of the balloon 20 described above. Note that the balloon 20 may be filled with gas except for the portion of the balloon 20 through which ultrasonic waves U are transmitted.

The bag 40 is disposed between the skin S above the blood vessel V and the portion of the presser 10 that presses the skin S above the blood vessel V. The skin S is pressed by the presser 10 through the bag 40. The internal pressure of the bag 40 is the pressure generated by the presser 10 and the skin S being pressed against each other, which corresponds to the pressure exerted on the blood vessel V when the skin S is sufficiently soft and the blood vessel V exists directly below the skin S. The liquid L2 in the bag 40 is in communication with the sensor 41 via a small tube. The sensor 41 detects the internal pressure of the bag 40 as the pressure exerted on the blood vessel V.

In this way, the pressure detector 11 detects the pressure generated between the skin S above the blood vessel V among the skin S pressed by the presser 10 and the portion of the presser 10 pressing on the skin S above the blood vessel V as the pressure exerted on the blood vessel V. In other words, by pressing on the skin S, the pressure detector 11 detects the pressure applied to a narrower area than the contact surface 20a as the arteriovenous pressure under the skin that is in contact with the area.

As illustrated in FIG. 2B, the pressure detectors 11 are set in a plurality of areas on the contact surface 20a. The pressure detectors 11 are arranged in a row in the X-axis direction along the skin S. In the present embodiment, the number of pressure detectors 11 is seven. This means that when the echo window 31 is brought into contact with the skin S, one of the seven pressure detectors 11 is positioned above the blood vessel V.

### [Processor and display]

As illustrated in FIG. 2A, the arteriovenous pressure measurement device 1 includes a processor 50 and a display 60, in addition to the presser 10 and the pressure detector 11 to which the probe 2 is attached. The probe 2 is electrically connected to and can communicate with the processor 50. The sensor 41 of the pressure detector 11 is also electrically connected to and can communicate with the processor 50.

The processor 50 converts an electrical signal transmitted from the probe 2 and pressure detector 11 and transmits the converted electrical signal to the display 60.

The display 60 displays a detection value of the sensor 41 based on the converted electrical signal. The pressure of the blood vessel V can also be displayed automatically by linking the detection value with a cross-sectional image of the measurement target T including the blood vessel V (artery or vein) using image recognition.

Next, the operation of the arteriovenous pressure measurement device 1 according to the present embodiment, that is, the arteriovenous pressure measurement processing, is described. Note that, when measuring a venous pressure, measurement is performed by compressing, for example, a leg vein of the measurement target T, while when measuring an arterial pressure, measurement is performed by compressing, for example, a brachial artery of the measurement target T.

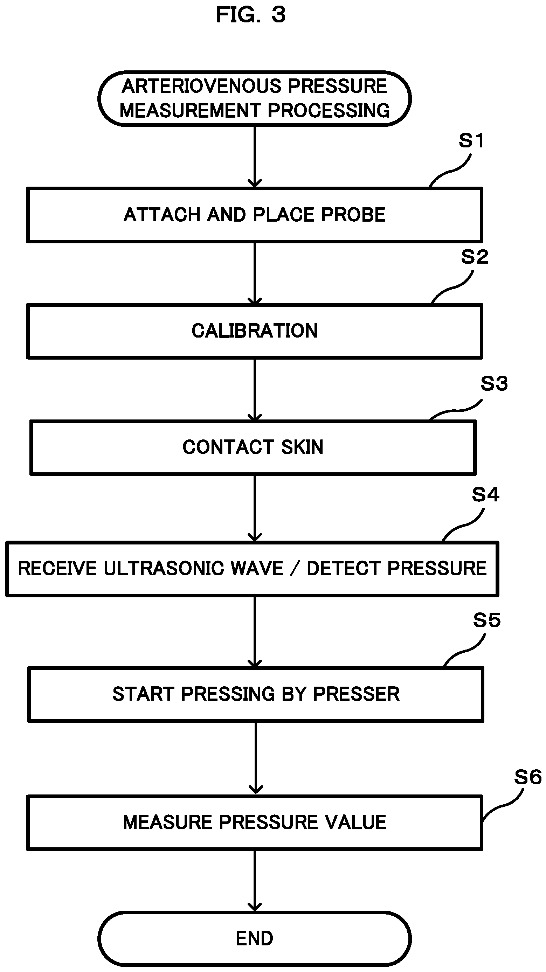

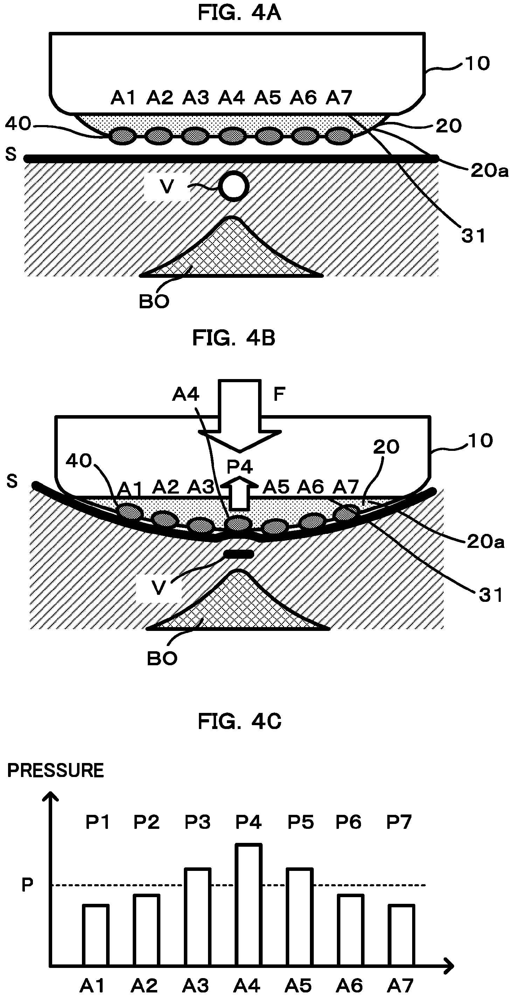

As illustrated in FIG. 3, an operator first attaches the probe 2 to the arteriovenous pressure measurement device 1 and places the arteriovenous pressure measurement device 1 and the probe 2 at the measurement site on the skin S (Step S 1). As illustrated in FIG. 4A, this places, on the skin, the presser 10 that presses on the skin S with the portion transmitting ultrasonic waves U from the probe 2 and the pressure detector 11 that detects the pressure exerted on the skin S above the blood vessel V among the skin S pressed by the presser 10 as the pressure on the blood vessel V. In other words, disposed on the skin S are: the presser 10 that has the balloon 20 and the bag 40 as members for transmitting ultrasonic waves U transmitted from the probe 2 and that is pressed in a direction where the ultrasonic waves U are transmitted in the contact surface 20a of those members, where the pressing force can be adjusted manually or by an adjustment mechanism; and the pressure detector 11 that is set on the contact surface 20a in a narrower area than the contact surface 20a and detects the pressure applied to the area.

The operator holds the arteriovenous pressure measurement device 1 and calibrates the sensor 41 for fine adjustment (Step S2). This calibration is performed by initializing (zero-setting) the pressure detected by the sensor 41 to zero in a state illustrated in FIG. 4A, that is, a state where the skin S is not pressed by the presser 10 (before the balloon 20 contacts the skin S). This allows accurate measurement of the vascular pressure with this sensor 41.

Thereafter, the operator brings the echo window 31 into contact with the skin S of the measurement target T (Step S3), and starts transmission and reception of ultrasonic waves U from the probe 2 and pressure detection by the pressure detector 11 (Step S4). As a result, a cross-sectional image of the measurement target T including the blood vessel V (refer to FIG. 2B) is displayed on the screen of the display 60. Such an image can be a brightness (B) mode image. Here, for example, as illustrated in FIGS. 4A and 4B, the seven bags 40 are denoted as bags A1 to A7 from left to right. The image displayed on the display 60 indicates the positional relationship between the bags A1 to A7 and the blood vessel V, that is, which bag 40 corresponds to the position of the blood vessel V. In FIGS. 4A and 4B, the position of the bag A4 corresponds to the position of the blood vessel V.

Thereafter, the operator starts pressing with the presser 10 (Step S5). As illustrated in FIG. 4A, the contact surface 20a of the balloon 20 is originally positioned so that the contact surface 20a protrudes slightly from the echo window 31, but when the push button 22 is pressed, the internal pressure of the balloon 20 is further increased as illustrated in FIG. 4B, allowing measurement to an even higher pressure range. This bulging or further bulging balloon 20 presses on the skin S. Since the outer surface of the balloon 20 filled with the liquid L1 presses on the skin S, the outer surface of the solid flexible member without the liquid L1 does not contact the skin S, the skin and subcutaneous tissues are not obstructed by a hard outer surface, and the skin S can be pressed with a precise fit between the outer surface of the balloon 20 and the outer surface of the skin S. The pressurization of the pressure in the balloon 20 necessary for the bulge of the balloon 20 through the echo window 31 does not have to be done by manual compression of the push button 22 by the operator, but the contact surface 20a may be caused to bulge by the inflation of the gas bag 25 by the adjustment mechanism. It can also be done by supplying pressurized water into the balloon 20 by the adjustment mechanism through a tube, not illustrated, connected to the balloon 20.

At this time, the degree of pressing the push button 22 is preferably adjusted so that the skin S is gradually pressed down. This allows the operator to confirm with a tomographic image on the display 60 how the blood vessel V is collapsed as the skin S is gradually pressed by the presser 10.

In parallel with the pressing by the presser 10, the detection value of the sensor 41 at the moment when the blood vessel V collapses, that is, the pressure value in the bag 40, is measured as the blood pressure value of the blood vessel V (Step S6). Here, a tomographic image of the blood vessel V based on ultrasonic waves U received by the probe 2 is displayed, and the pressure applied to the blood vessel V is detected by the pressure detector 11 in a state where the skin S is pressed with the presser 10. Here, the collapse of the blood vessel V means that a blood vessel V is pressed from the surface, the front side of the blood vessel V is compressed and approaches the back side of the blood vessel V, blood in the blood vessel V moves elsewhere and blood is pushed out from inside the blood vessel V, causing the walls of the front side and the back side of the blood vessel V to stick together to crush the blood vessel V. In the present embodiment, collapse of the blood vessel V occurs when the pressing force of the balloon 20 is equal to or slightly greater than the pressure of the blood in the blood vessel V.

If the head 2a of the probe 2 that is the ultrasonic wave U transmitting/receiving surface (oscillation surface) is directly pressed against the skin S to collapse the blood vessel V, since the head 2a of the probe 2 is hard, the thin and low-pressure blood vessel V is crushed by compression by the head 2a, and the blood vessel V cannot be recognized as the blood vessel V and may be overlooked. Since the balloon 20 is softer than the head 2a of the probe 2, the balloon 20 hardly crushes the blood vessel V directly under skin S. Operating the push button 22 can collapse the blood vessel V with a constant pressing force. This prevents inadvertent crushing of the blood vessel V.

For example, as illustrated in FIG. 4B, when the blood vessel V to be measured is directly above a bone BO, a pressure imbalance occurs in the subcutaneous tissue due to the bone BO located behind the blood vessel V, and the blood vessel V collapses with pressure P smaller than the actual internal pressure of the blood vessel V. In this case, as illustrated in FIG. 4C, the pressure in the balloon 20 is the average pressure P due to the reaction force received from the skin S, while the pressures of the bags A1 to A7 are P1 to P7 due to the reaction force received from the skin S directly below the bags A1 to A7. When the blood vessel V is directly above a hard object such as a protrusion of the bone BO, the blood pressure of the blood vessel V is measured lower than the actual internal pressure of the blood vessel V when the blood pressure of the blood vessel V is determined based on the pressure inside the balloon 20. On the other hand, focusing on the bag 40 (A4), when the pressure P4 within the bag A4 is lower than the internal pressure of the blood vessel V, the front surface of the blood vessel V will not come in contact with the back surface of the blood vessel V, without causing the blood vessel V to be crushed. Furthermore, as the pressure P4 within the bag A4 increases, and when the pressure becomes equal to or slightly higher than the internal pressure of the blood vessel V, the blood vessel V is crushed. The pressure P4 in the bag A4 in this state is an accurate representation of the blood pressure of the blood vessel V. Therefore, the pressure P4 when the blood vessel V is crushed is measured as the blood pressure of the blood vessel V.

After the blood pressure measurement, that is, after completion of Step S5, the pressing force by the presser 10 is released and the arteriovenous pressure measurement device 1 is removed from the skin S to end the arteriovenous pressure processing.

In this way, the arteriovenous pressure measurement device 1 of the present embodiment can measure a venous pressure at any of various sites noninvasively within a few seconds, and the measurement can be repeated easily. Furthermore, unlike observation of a vein with a hard probe in general echo testing, this test method observes a superficial vein with a soft, flexible contact area in a state where the pressure of the balloon 20 made of flexible material is not increased, so that the superficial vein is not easily crushed when the arteriovenous pressure measurement device 1 makes contact. This increases the likelihood that even a low venous pressure can be measured accurately.

When measuring arterial pressure, a diastolic pressure is pressure at the time when the arterial blood flow ceases to be continuous as the pressure of the balloon 20 increases, and a systolic pressure is when the arterial blood flow stops completely. Thus, the systolic and diastolic pressures of the artery are measured by contrasting the pressure of the balloon 20 of the presser 10 with the flow of blood in the artery.

The moment when the arterial blood flow ceases to be continuous (diastolic pressure) and the moment when the blood flow completely stops (systolic pressure) can also be determined by observing a moving image of an artery (the appearance of beating) in an image on the display 60. Furthermore, by recognizing and determining this image by a computer using image processing technology of the computer and linking the data with the measurement value of the sensor 41, an even more accurate measurement of systolic and diastolic arterial pressures can be performed. For example, a machine learner may be used to learn with a moving image of the blood vessel V in a tomographic image, identify the timing, and determine the pressure value at the timing as the systolic or diastolic pressure.

In addition, an even more accurate measurement of systolic and diastolic arterial pressures is possible by detecting an arterial blood flow using a Doppler method such as color Doppler, power Doppler, pulse wave Doppler, or continuous wave Doppler, recognizing the moment when the arterial blood flow is no longer continuous (diastolic pressure) and the moment when the blood flow stops completely (systolic pressure) by the computer image processing technology and computer analysis of the blood flow, and linking the data to the measurement value by the pressure sensor. Such recognition can also be achieved by machine learning using a large number of images by a machine learner.

The two-dimensional shape and motion of the measurement target T can be directly observed by using a B-mode where an ultrasonic beam is electronically scanned to produce a still or moving image of the two-dimensional distribution of echo intensity. However, the periodicity of motion cannot be observed in an instantaneous B-mode image. Therefore, a method to obtain the periodicity of the beating motion by computer-processing a plurality of frames of the B-mode images by an image processor, is used to find the periodicity of the motion. The diastolic and systolic blood pressures of an artery can be measured based on the pressure value detected by the pressure detector 11 of the arteriovenous pressure measurement device 1 and the periodicity of the beating motion.

Note that which bag 40 of the bags A1 to A7 is located on the skin S above the blood vessel V may be automatically determined based on data of a tomographic image by an ultrasonic echo, and a pressure value of the determined bag 40 may be automatically calculated as a blood pressure value.

Although the present embodiment illustrates the blood pressure measurement of a blood vessel V above a bone BO, this arteriovenous pressure measurement device 1 allows accurate blood pressure measurement of a blood vessel V above a projection of a bone BO as well as accurate blood pressure measurement of a blood vessel V above soft tissues. If a blood vessel V is located close to the skin S, accurate blood pressure measurement is possible no matter what type of tissue is under the blood vessel V. If the echo image is not automatically calculated using image recognition or machine learning, the relationship between the bags A1 to A7 in the echo image and the blood vessel V is recognized by the naked eyes, and the pressure in the bag 40 directly above the blood vessel V is measured as the pressure of the blood vessel V to be measured.

### Embodiment 2

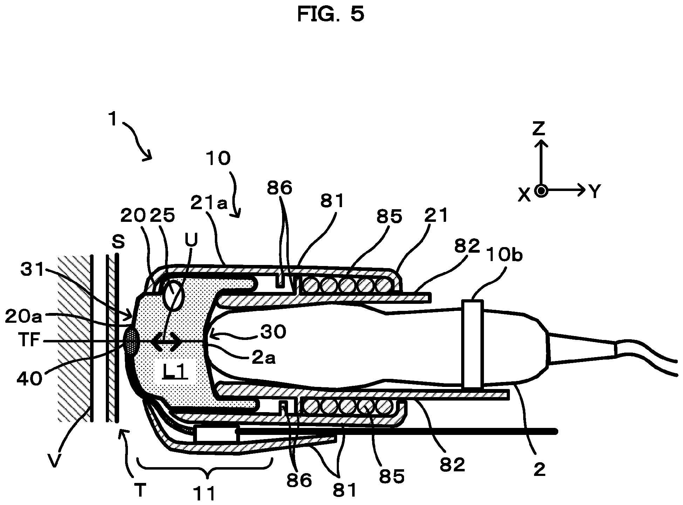

Next, Embodiment 2 of the present disclosure is described with reference to FIG. 5. In Embodiment 1 above, the operator holds the arteriovenous pressure measurement device 1, presses it against the measurement target T, and pushes the push button 22 onto the balloon 20 to cause the balloon 20 to bulge toward the skin S side. In other words, the arteriovenous pressure measurement device 1 according to Embodiment 2 automatically causes the balloon 20 to bulge toward the skin S side.

The arteriovenous pressure measurement device 1 according to Embodiment 2 is the same as the arteriovenous pressure measurement device 1 according to Embodiment 1 above in that the arteriovenous pressure measurement device 1 has a presser 10 and a pressure detector 11. The presser 10 includes a balloon 20 and a housing 21, and the pressure detector 11 includes a bag 40 and a sensor 41 in the same way as the arteriovenous pressure measurement device 1 according to Embodiment 1 above.

As illustrated in FIG. 5, in the arteriovenous pressure measurement device 1 according to Embodiment 2, the housing 21 is equipped with a cover 21a as an attachment, while an extension 21b is not provided. The cover 21a includes a bottomed cylindrical outer member 81 and a cylindrical inner member 82 introduced inside the outer member 81. An echo window 31 is set at the -Y side end of the outer member 81, and the balloon 20 is housed at the -Y side of the outer member 81. The probe 2 is introduced in the inner member 82, and the head opening 30 is provided at the -Y side end of the inner member 82. The inner member 82 is provided with a belt 10b, and the probe 2 is secured to the inner member 82 by the belt 10b.

An expandable and contractible gas bag 25 is contained inside the balloon 20. Although not illustrated in FIG. 5, the configuration for expanding and contracting the gas bag 25 is the same as that of the arteriovenous pressure measurement device 1 according to Embodiment 1 above.

Also, attached to the inner wall of the outer member 81 is a ball spline 85 as an advancing/retracting mechanism that moves the probe 2 in and out with respect to the ultrasonic wave U transmitting portion of the balloon 20. By rotating the ball spline 85, the inner member 82 can move freely in and out with respect to the outer member 81. This allows the probe 2 to move in and out with respect to the presser 10. Stoppers 86 are provided on the inner wall of the outer member 81 and the outer wall of the inner member 82, respectively, to prevent the inner member 82 from being pulled out from the outer member 81. In other words, the advancing/retracting mechanism can be a pressing mechanism that presses the probe 2 on the balloon 20 through the head opening 30 to cause the contact surface 20a of the balloon 20 to bulge through the first opening.

In the arteriovenous pressure measurement device 1 of Embodiment 2, the ball spline 85 pushes the inner member 82 into the outer member 81, thereby pushing the probe 2 held by the inner member 82 against the balloon 20 of the outer member 81. This increases the internal pressure of the balloon 20 and enables measurement of high venous or arterial pressure. A portion of the balloon 20 that bulges through the echo window 31 (contact surface 20a) presses on the skin S to enable blood pressure measurement.

This arteriovenous pressure measurement device 1 can be made more compact compared to the arteriovenous pressure measurement device 1 according to Embodiment 1, since the arteriovenous pressure measurement device 1 does not need to be equipped with the push button 22.

### Embodiment 3

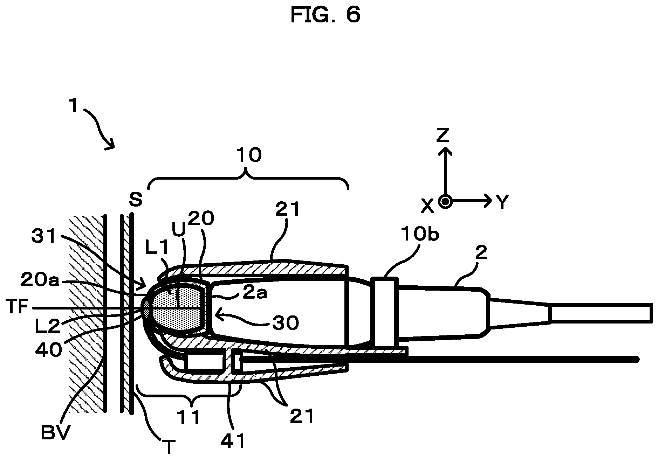

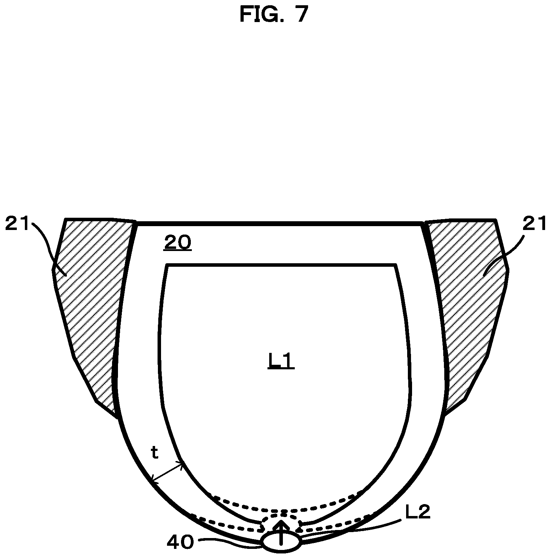

Next, Embodiment 3 of the present disclosure is described with reference to FIGS. 6 and 7. The arteriovenous pressure measurement device 1 according to Embodiment 3 differs in the configuration of the balloon 20 that constitutes the presser 10. As illustrated in FIG. 6, also in Embodiment 3, the balloon 20 is filled with a liquid L1, and the mechanism for filling the liquid L1 is the same as in the arteriovenous pressure measurement device 1 according to Embodiment 1 above. The probe 2 is attached to the housing 21 and secured with the belt 10b, allowing ultrasonic waves U to transmit the balloon 20.

As illustrated in FIG. 7, the balloon 20 is configured so that the wall thickness t of the portion constituting the outer surface that bulges through the echo window 31 is the thinnest at a portion where pressure is detected by the pressure detector 11, that is, a portion where the bag 40 is provided, and gradually thickens from the bag 40 toward the edge of the echo window 31. As a result, the wall thickness t of the balloon 20 is thinner in the vicinity of the bag 40, making it easier to deform with small force, as illustrated by the dashed line, and to compress the skin S above the blood vessel V with a soft touch. Thus, low venous pressure can be measured accurately. On the other hand, when the balloon 20 is compressed with strong force using the presser 10, the internal pressure of the balloon 20 gradually increases as the balloon 20 is gradually crushed from the tip (the portion of the bag 40), but the thick portion of the balloon 20 receives this increase in the internal pressure, enabling accurate measurement of high arterial and venous pressure.

The wall thickness t of the balloon 20 is thickened toward the housing 21, preventing the portion of the balloon 20 other than the periphery of the bag 40 from deforming outward.

### Embodiment 4

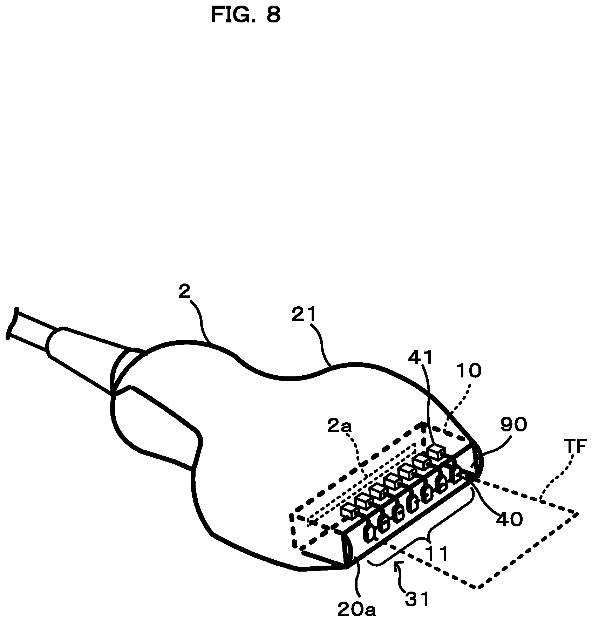

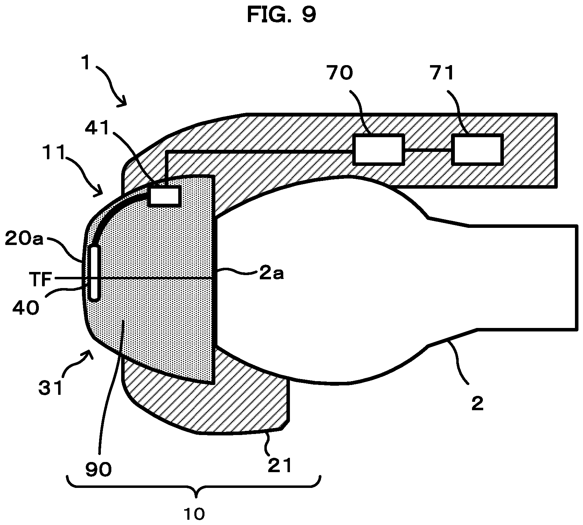

Next, Embodiment 4 of the present disclosure is described with reference to FIGS. 8 and 9. The arteriovenous pressure measurement device 1 according to Embodiment 4 above has the probe 2 as a separate unit and uses the probe 2 by mounting the probe 2. The arteriovenous pressure measurement device 1 according to Embodiment 4 is integrated with the probe 2. That is, the arteriovenous pressure measurement device 1 according to Embodiment 4 incorporates a probe 2 that transmits and receives ultrasonic waves U.

As illustrated in FIGS. 8 and 9, the presser 10 has a housing 21 and an elastic member 90. The elastic member 90 is made of a material that transmits ultrasonic waves U and is provided at the outlet (header 2a) of ultrasonic waves U of the probe 2. The elastic member 90 is made of gel or silicone, but is not necessarily limited to gel or silicone as long as the elastic member transmits ultrasonic waves U.

In Embodiment 4, the pressure detector 11 is embedded in the elastic member 90. More specifically, the pressure detector 11 is embedded in the portion of the elastic member 90 that is in contact with the skin S, that is, directly below the contact surface 20a. The pressure detector 11 includes a bag 40 and a sensor 41 in the same way as the arteriovenous pressure measurement device 1 according to Embodiment 4 above.

The arteriovenous pressure measurement device 1 according to Embodiment 4 above includes a signal processor 70 and a wireless communicator 71. The signal processor 70 converts a signal indicating pressure detected by the pressure detector 11 into data that can be transmitted by the wireless communicator 71. The wireless communicator 71 transmits the data converted by the signal processor 70 to a communication terminal, not illustrated, through a wireless communication. The communication terminal displays the pressure value detected by the pressure detector 11. This wireless communication may be performed by any other type of measurement device described in FIGS. 1A to 7 above. Note that the communication terminal may be a smartphone or a computer such as a tablet. The method of the wireless communication is not limited, and various methods such as Wi-Fi, Bluetooth (registered trademark), and/or the like may be used. The operability of the arteriovenous pressure measurement device 1 (probe 2) can be improved if data can be transmitted via such a wireless communication.

Note that, in the pressure detector 11, a piezoelectric element can be embedded in the elastic member 90 instead of the bag 40 as long as a tomographic image of the blood vessel V can be acquired. The electrical signal indicating the pressure from the skin S detected by the piezoelectric element is output to the signal processor 70. The piezoelectric element is desirably as thin as possible so that the probe 2 can acquire a tomographic image of the blood vessel V. The piezoelectric element may be slightly displaced from the surface through which ultrasonic waves U are transmitted. In this case, piezoelectric elements may be introduced on both sides of the elastic member 90 by displacing the piezoelectric elements from the ultrasonic wave U transmitting surface, and the average of the detection signals of the piezoelectric elements introduced on both sides may be adopted as the pressure value.

### Embodiment 5

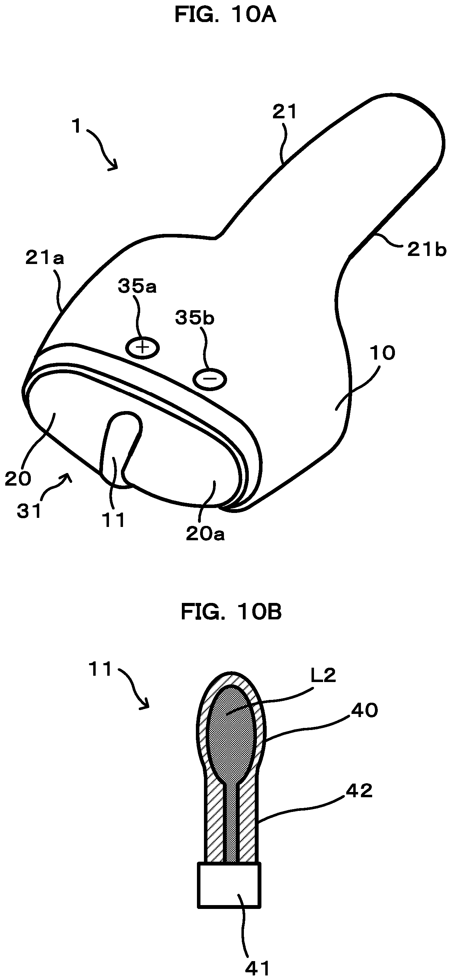

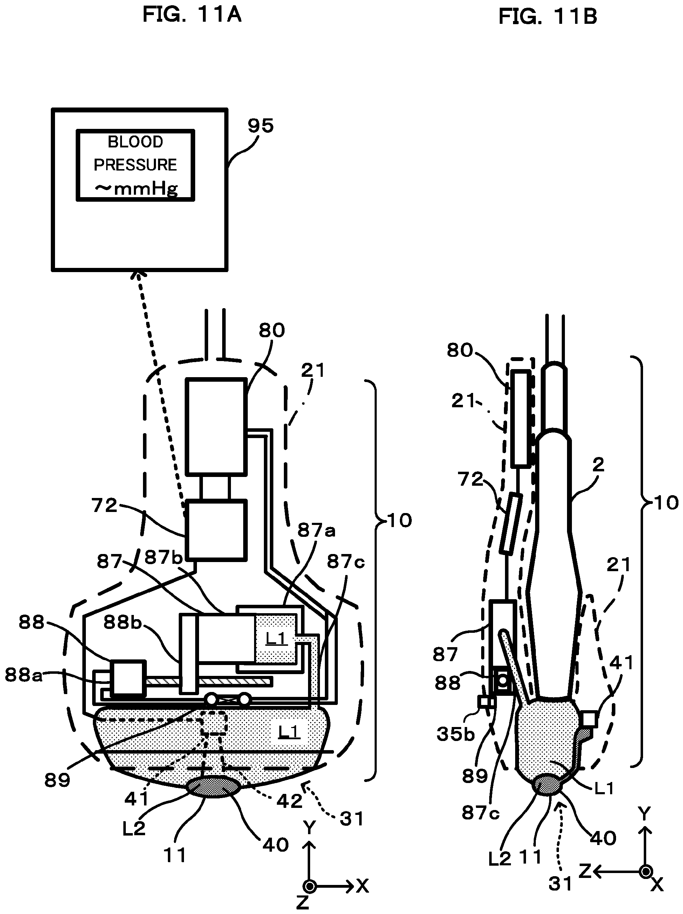

Next, Embodiment 5 of the present disclosure is described with reference to FIGS. 10A, 10B, 11A, and 11B. While the arteriovenous pressure measurement device 1 according to Embodiment 2 above has the ball spline 85 as an advancing/retracting mechanism, the arteriovenous pressure measurement device 1 according to Embodiment 5 has a drive mechanism that can freely cause the contact surface 20a to bulge.

As illustrated in FIG. 10A, the arteriovenous pressure measurement device 1 according to Embodiment 5 is the same as the arteriovenous pressure measurement device 1 according to Embodiment 5 above in that the arteriovenous pressure measurement device 1 includes a presser 10 and a pressure detector 11. The housing 21 includes a cover 21a and an extension 21b in the same way as the arteriovenous pressure measurement device 1 according to Embodiment 5 above. The cover 21a is also provided with a portion of the balloon 20 that bulges to provide a contact surface 20a, and the contact surface 20a is provided with the bag 40 of the pressure detector 11 in the same way as the arteriovenous pressure measurement device 1 according to Embodiments 1 to 4 above.

As illustrated in FIG. 10A, the arteriovenous pressure measurement device 1 according to Embodiment 5 of the present disclosure includes one pressure detector 11, but the arteriovenous pressure measurement device 1 may include a plurality of pressure detectors 11. As illustrated in the expanded diagram of FIG. 10B, in the pressure detector 11, the bag 40 and the sensor 41 are connected by a conduit 42 extending along the balloon 20, and the pressure generated in the bag 40 can be measured by the sensor 41. The conduit 42 is set along the balloon 20 and is made of an elastic material. In order to accurately detect the pressure inside the bag 40 by the sensor 41, the thickness of the elastic material constituting the conduit 42 is thicker than the thickness of the elastic material constituting the bag 40.

As illustrated in FIGS. 11A and 11B, the presser 10 includes a feeder 87 for the balloon 20 and an actuator 88 that drives the feeder 87 by an input operation of an operator. The feeder 87 includes a syringe 87a, a plunger 87b, and a tube 87c. The interior of the syringe 87a is in communication with the interior of the balloon 20 through the tube 87c, which is filled with a liquid L1. As the plunger 87b moves, the liquid L1 moves back and forth between the syringe 87a and the balloon 20. This allows to control the amount of bulge of the contact surface 20a of the balloon 20.

The actuator 88 includes a motor 88a and a feed screw 88b connected to the rotating shaft of the motor 88a. The plunger 87b is connected to the feed screw 88b, and when the motor 88a rotates, the feed screw 88b rotates, thereby moving the plunger 87b.

As illustrated in FIGS. 11A and 11B, the arteriovenous pressure measurement device 1 embeds a battery 80. The battery 80 is connected to the motor 88a of the actuator 88 via a switch 89. The cover 21a is provided with a plus button 35a and a minus button 35b. When the plus button 35a or minus button 35b is pressed by an input operation of the operator, the switch 89 is closed and power from the battery 80 is supplied to the motor 88a. When the plus button 35a is pressed, the motor 88a rotates forward and moves the plunger 87b in a direction of pushing out the liquid L1. This can increase the amount of bulge of the contact surface 20a of the balloon 20. When the minus button 35b is pressed, the motor 88a rotates backward to move the plunger 87b in a direction of returning the liquid L1 to the feeder 87. This can reduce the amount of bulge of the contact surface 20a of the balloon 20. This action can adjust the pressing force of the presser 10.

In this way, the feeder 87 can supply the liquid L1 to the balloon 20, and the actuator 88 drives the feeder 87 to supply the liquid L1 to the balloon 20 by an input operation of the operator.

The arteriovenous pressure measurement device 1 includes a microcomputer 72. The microcomputer 72 embeds a signal processor 70 and a wireless communicator 71, as illustrated in FIG. 9. The microcomputer 72 is connected to the sensor 41 through I²C (Inter-Integrated Circuit) communication or other means. The communication method used for this connection is not necessarily limited to I²C communication, but can employ any of various communication methods, for example, Universal Asynchronous Receiver/Transmitter (UART) communication and Clocked Serial Interface (Serial Peripheral Interface) (CSI (SPI)) communication. The signal processor 70 generates information indicating the pressure detected by the pressure detector 11, that is, the sensor 41, and transmits the generated information to a mobile terminal 95, such as a smartphone, via a wireless communication, for example, Wi-Fi. The mobile terminal 95 receives the information transmitted via a wireless communication and displays the blood pressure based on the received information.

The method of measuring blood pressure using the arteriovenous pressure measurement device 1 is the same as the arteriovenous pressure measurement device 1 according to Embodiment 5 above. Basically, the plus button 35a is pressed with the bag 40 pressed against the skin S above the blood vessel V, the actuator 88 is driven to supply the liquid L1 to the balloon 20 from the feeder 87, the minus button 35b is pressed as necessary to adjust the pressing force of the presser 10, and blood pressure is measured while viewing the tomographic waveform of the ultrasonic waves U transmitted and received by the probe 2.

### Embodiment 6

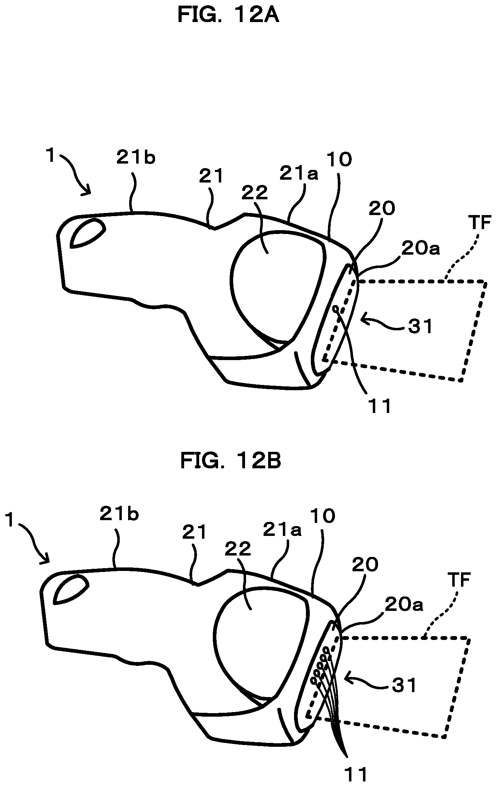

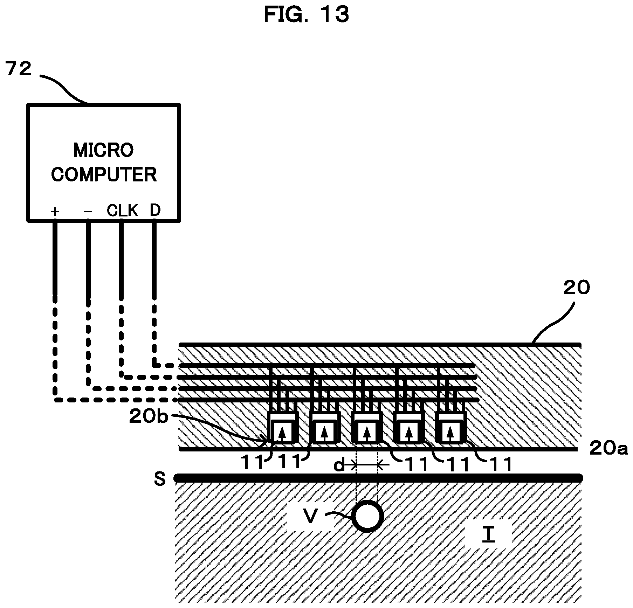

Next, Embodiment 6 of the present disclosure is described with reference to FIGS. 12A to 12D and FIG. 13. As illustrated in FIG. 12A, the arteriovenous pressure measurement device 1 according to Embodiment 6 is the same as the arteriovenous pressure measurement device 1 according to Embodiment 1 above in that the arteriovenous pressure measurement device 1 includes a balloon 20, a housing 21, and a push button 22. The arteriovenous pressure measurement device 1 also includes a presser 10 and a pressure detector 11 in the same way as the arteriovenous pressure measurement device 1 according to Embodiments 1 to 5 above. The arteriovenous pressure measurement device 1 also presses the skin S with the contact surface 20a that is the outer surface of the balloon 20 that bulges through the echo window 31 in the same way as the arteriovenous pressure measurement device 1 according to Embodiment 1 above.

The pressure detector 11 constituting the arteriovenous pressure measurement device 1 according to Embodiments 1 to 5 above includes a bag 40 placed on the ultrasonic wave U scanning plane (transmission surface) TF. The pressure detector 11 constituting the arteriovenous pressure measurement device 1 according to Embodiment 6 is a pressure sensor that detects pressure exerted in one direction.

The pressure detector 11 has a size equivalent to or smaller than the diameter of the blood vessel V under the skin S, similar to the bag 40 according to Embodiment 6 above (see FIG. 13). As illustrated in FIG. 12A, the pressure detector 11 (pressure sensor) is embedded in the area directly below the contact surface 20a of the balloon 20 that bulges through the echo window 31. The pressure detector 11 detects the pressure applied to the area from the opposite direction of the pressing direction of the presser 10 as the pressure exerted on the blood vessel.

More precisely, as illustrated in FIG. 12A, the pressure detector 11 is displaced to one side of the normal direction of the scanning plane TF from the scanning plane TF through which ultrasonic waves U are transmitted. The pressure detector 11 detects the pressure exerted in the opposite direction of the pressing direction of the presser 10. In this case, the position of the pressure detector 11 must be aligned with the skin S above the blood vessel V. The pressure detector 11 is zeroed before the pressure detector 11 is brought into contact with the skin S. When there is only one pressure detector 11, the detection value of the pressure detector 11 needs to be zeroed (initialized) each time the posture of the arteriovenous pressure measurement device 1 changes with respect to the measurement target T.

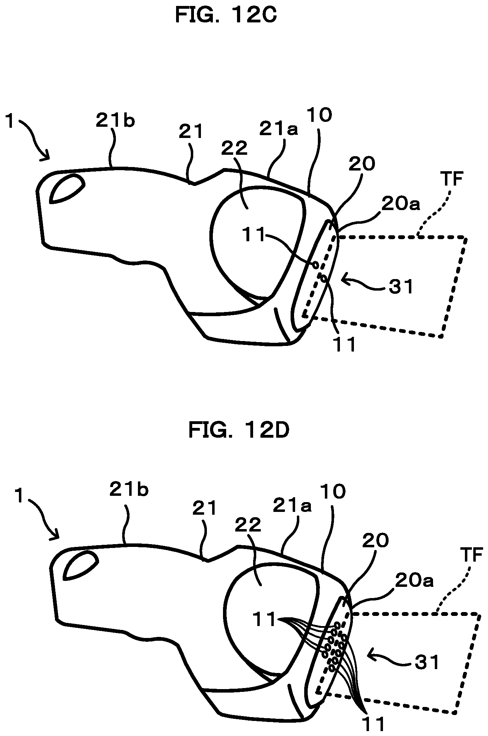

As illustrated in FIG. 12B, the pressure detectors 11 may be arranged in a row at positions displaced from the scanning plane TF to one side of the normal direction of the scanning plane TF. In this case, the detection value of the pressure detector 11 that is in contact with the skin S above the blood vessel V is employed (see FIG. 13). As illustrated in FIG. 12C, the pressure detectors 11 may be arranged one at positions displaced from the scanning plane TF, through which ultrasonic waves U are transmitted, to each of both sides of its normal direction. In this case, the blood pressure can be measured based on the average of the detection values of the pressure detectors 11 on both sides of the normal direction of the scanning plane TF. As illustrated in Fig. 12D, the pressure detectors 11 may be arranged in a row at positions displaced from the scanning plane TF, through which ultrasonic waves U are transmitted, on each of both sides of its normal direction. In this case, the blood pressure can be measured based on the average of the detection values of the pressure detectors 11 in contact with the skin S above the blood vessel V.

FIG. 13 illustrates the arrangement of the pressure detectors 11 in the configuration illustrated in FIG. 12B. As illustrated in FIG. 13, the pressure detectors 11 are embedded in the vicinity of the contact surface 20a in the wall 20b of the balloon 20. The pressure detectors 11 are arranged in a row and are capable of detecting pressure received from outside the contact surface 20a, that is, the pressure exerted in the direction of the arrows. Although the pressure detectors 11 are in contact with the skin S separated by the wall 20b (front rubber), the thickness of the wall 20b is extremely thin and flexible, so the influence of the front rubber on the detection value of the pressure detectors 11 is negligible.

If the detection value of the pressure detectors 11 is affected by the curvature state of the front rubber, the detection value of the pressure detectors 11 may be corrected according to the curvature state. In this case, the curvature state of the front rubber is obtained by image processing the tomographic image. This image processing may be performed by a deep learner that performs deep learning.

As in Embodiment 5 above, the pressure detector 11 (pressure sensor) can communicate with the microcomputer 72 by I²C communication or the like. In the case of I²C communication, the microcomputer 72 is the master and the pressure detector 11 is the slave, and both are connected by four cables. Of the four cables, two are power cables (+ and -), one is a clock signal line, and the remaining one is a data signal sensor. Note that the method of communication between the microcomputer 72 and the pressure detector 11 is not limited to I²C communication as described above.

As explained in detail above, according to the above embodiments, while pressing on the skin S with the presser 10 to crush the blood vessel V under the skin S, the pressure detector 11 can detect the pressure generated in the area where the skin S above the blood vessel V and the presser 10 come into contact, that is, the pressure close to the actual pressure exerted on the blood vessel V, instead of the average pressure exerted on the blood vessel V and its surrounding tissues as a whole pressed by the presser 10. This allows non-invasive and more accurate measurement of arteriovenous pressure.

Various configurations can be employed for the configuration of the presser 10 that presses on the skin S, as described in the above embodiments: the one pressing by a push button 22 or a gas bag 25 with gas sealed inside; the one including an advancing/retracting mechanism that advances and retracts the probe 2; and the one including a drive mechanism that can automatically change the amount of bulge. The presser 10 can include at least one of these components.

In other words, the presser 10 can be configured to press on the skin S by increasing the internal pressure of the balloon 20 by compressing the balloon 20, or by increasing the internal pressure of the balloon 20 by supplying gas or liquid into the balloon 20.

In the above embodiments, the presser 10 is assumed to be flexible. However, the presser 10 is not limited to this. The presser 10 may be made of plastic with low flexibility.

The arteriovenous pressure measurement device 1 according to the above embodiments includes, as main components, a presser 10 that presses on the skin S and a pressure detector 11. As explained in Embodiments 1 to 3, 5, and 6 above, the probe 2 and the presser 10 may be separate units, or as explained in Embodiment 4 above, the probe 2 and the presser 10 may be integrated into a single structure. To reduce the cost of the device, the probe 2 and the presser 10 are preferably separated.

The arteriovenous pressure measurement device 1 according to the above embodiments can accurately measure blood pressure as long as the blood vessel V is close to the skin S. Blood pressure can be measured at various sites, such as the wrist, arm, or leg.

In the above embodiments, the pressure detectors 11 are arranged in a single row. However, the present disclosure is not limited to this. There may be only one pressure detector 11. Even when there is only one pressure detector 11, measurement can be performed by positioning the pressure detector 11 on the skin S above a blood vessel V while viewing a tomographic image displayed on the display 60. Alternatively, the pressure detectors 11 may be arranged in a plurality of rows.

In the above embodiments, the probe 2 is the probe of an ultrasonic echo device. However, the present disclosure is not limited to this. For example, the probe of an ultrasonic blood flow meter may be used as the probe 2.

The present disclosure is not limited to the shape of the probe 2. For example, a pencil-type pulse Doppler probe can be adopted. The arteriovenous pressure measurement device 1 attached to the pencil-type probe gradually compresses an artery and detects the pressure when the arterial blood flow is interrupted. The pressure value detected by the pressure detector 11 upon the transient interruption is the diastolic pressure, and the pressure value detected by the pressure detector 11 upon complete interruption is the systolic pressure.

The foregoing describes some example embodiments for explanatory purposes. Although the foregoing discussion has presented specific embodiments, persons skilled in the art will recognize that changes may be made in form and detail without departing from the broader spirit and scope of the invention. Accordingly, the specification and drawings are to be regarded in an illustrative rather than a restrictive sense. This detailed description, therefore, is not to be taken in a limiting sense, and the scope of the invention is defined only by the included claims, along with the full range of equivalents to which such claims are entitled.

This application claims the benefit of Japanese Patent Application No. 2022-26161 filed on February 22, 2022, the entire disclosure of which is incorporated by reference herein.

### Industrial Applicability

The present disclosure can be applied to arterial and venous measurements.

### Reference Signs List

- 1: Arteriovenous pressure measurement device

- 2: Probe
- 2a: Head
- 10: Presser
- 10a: Recess
- 10b: Belt
- 11: Pressure detector
- 20: Balloon
- 20a: Contact surface
- 20b: Wall
- 21: Housing
- 21a: Cover
- 21b: Extension
- 21c: Pincer piece
- 22: Push button
- 23: Tube
- 24: Liquid volume adjustment valve
- 25: Gas bag
- 26: Tube
- 27: Pressure adjustment valve
- 30: Head opening
- 31: Echo window
- 32: Button attachment opening
- 35a: Plus button
- 35b: Minus button
- 40: Bag
- 41: Sensor
- 42: Conduit
- 50: Processor
- 60: Display
- 70: Signal processor
- 71: Wireless communicator
- 72: Microcomputer
- 80: Battery
- 81: Outer member
- 82: Inner member
- 85: Ball spline
- 86: Stopper
- 87: Feeder
- 87a: Syringe
- 87b: Plunger
- 87c: Tube
- 88: Actuator
- 88a: Motor
- 88b: Feed screw
- 89: Switch
- 90: Elastic member
- 95: Mobile terminal
- BO: Bone
- F: Floor
- L1, L2: Liquid
- S: Skin
- U: Ultrasonic wave
- V: Blood vessel
- T: Measurement target
- TF: Scanning plane

## Claims

1. An arteriovenous pressure measurement device comprising:
a presser that comprises a member that transmits ultrasonic waves that have been transmitted from a probe scanning around one direction and that have been reflected and received by the probe, wherein the presser is pressed, in a direction where the ultrasonic waves are transmitted, at a contact surface created on an outer surface of the member and intersected by a scanning plane made by scanning of the ultrasonic waves by the probe, wherein the pressing force of the presser is adjustable manually or by an adjustment mechanism; and
a pressure detector that is set in a narrower area than the contact surface on or directly below the contact surface and detects pressure applied to the area by pressing on a skin as arteriovenous pressure under the skin that the area contacts.

2. The arteriovenous pressure measurement device according to claim 1, wherein a size of the area is equivalent to or less than a diameter of a blood vessel under the skin that the area contacts.

3. The arteriovenous pressure measurement device according to claim 1 or 2, wherein the pressure detector comprises:
a flexible bag that is set in the area, made of a material transmitting the ultrasonic waves, and filled with a fluid transmitting the ultrasonic waves; and
a sensor that detects an internal pressure of the bag as pressure exerted on a blood vessel.

4. The arteriovenous pressure measurement device according to claim 1 or 2, wherein the pressure detector comprises a pressure sensor that is embedded in the area of the member directly below the contact surface and detects pressure applied to the area from an opposite direction of a pressing direction of the presser as pressure exerted on a blood vessel.

5. The arteriovenous pressure measurement device according to any one of claims 1 to 4, wherein the pressure detector is displaced from the scanning plane to one or both sides of a normal direction of the scanning plane.

6. The arteriovenous pressure measurement device according to any one of claims 1 to 5, comprising a plurality of the pressure detectors.

7. The arteriovenous pressure measurement device according to claim 6, wherein the pressure detectors are arranged in one or more rows.

8. The arteriovenous pressure measurement device according to any one of claims 1 to 7, wherein the presser comprises:
a flexible container that is made of a flexible material that transmits the ultrasonic waves and contains a fluid that transmits the ultrasonic waves; and
a housing that includes:
a first opening that houses the flexible container, wherein the flexible container bulges in a direction of pressing on the skin to create the contact surface; and
a second opening that opposes the first opening and allows ultrasonic waves transmitted from the probe to enter through the second opening.

9. The arteriovenous pressure measurement device according to claim 8, wherein
the housing comprises a third opening, and
the presser comprises an external manipulator that causes the contact surface of the flexible container to bulge through the first opening when an operator manually presses on the flexible container through the third opening.

10. The arteriovenous pressure measurement device according to claim 8, wherein the presser comprises a pressing mechanism that presses the probe against the flexible container through the second opening to cause the contact surface of the flexible container to bulge through the first opening.

11. The arteriovenous pressure measurement device according to claim 8, wherein the presser comprises:
a feeder capable of supplying the fluid to the flexible container; and
an actuator that causes, through an input operation by an operator, the feeder to supply the fluid to the flexible container to cause the contact surface of the flexible container to bulge.

12. The arteriovenous pressure measurement device according to any one of claims 8 to 11, wherein the flexible container that bulges through the first opening has a wall thickness that is thinnest at a portion where the pressure detector is provided and gradually thickens toward an edge of the first opening.

13. The arteriovenous pressure measurement device according to any one of claims 8 to 12, wherein the housing comprises an attachment that mounts the probe so that a pressing direction of the presser is aligned with the scanning plane.

14. The arteriovenous pressure measurement device according to any one of claims 8 to 13, wherein the housing comprises a gripper to be held by an operator.

15. The arteriovenous pressure measurement device according to any one of claims 1 to 7, comprising the probe, wherein
the presser comprises an elastic member provided at an outlet of the ultrasonic waves of the probe and transmits the ultrasonic waves, and
the pressure detector is embedded in the elastic member.

16. The arteriovenous pressure measurement device according to any one of claims 1 to 15, comprising:
a wireless communicator that transmits information indicating pressure detected by the pressure detector via a wireless communication; and
a display that receives information transmitted by the wireless communicator and displays the received information.

17. A method for measuring arteriovenous pressure, the method using an arteriovenous pressure measurement device that includes a presser that comprises a member that transmits ultrasonic waves that have been transmitted from a probe scanning around one direction and have been reflected and received by the probe, wherein the presser is pressed, in a direction where the ultrasonic waves are transmitted, at a contact surface created on an outer surface of the member and intersected by a scanning plane made by scanning of the ultrasonic waves by the probe, wherein the pressing force of the presser is adjustable manually or by an adjustment mechanism; and a pressure detector that is set in a narrower area than the contact surface on or directly below the contact surface and detects pressure applied to the area by pressing on a skin as arteriovenous pressure under the skin that the area contacts,
the method comprising:
disposing the arteriovenous pressure measurement device so that the area is in contact with the skin;
initializing pressure to be detected by the pressure detector in a state where the presser is not pressing on the skin;
starting to transmit and receive the ultrasonic waves by the probe;
pressing on the skin by the presser;
displaying a tomographic image of a blood vessel based on the ultrasonic waves received by the probe; and
detecting an arteriovenous pressure exerted on the blood vessel under the skin in a state where the presser is pressing on the skin by the pressure detector.
